# EUROPÄISCHE PATENTSCHRIFT

(11) **EP 2 043 977 B1**
(45) Veröffentlichungstag und Bekanntmachung des Hinweises auf die Patenterteilung: **10.10.2012**
(21) Anmeldenummer: 07787455.0
(22) Anmeldetag: 12.07.2007
(51) Int. Cl.: C07C 7/08, C07C 11/167, C10G 7/08

(54) **VERFAHREN ZUR AUFTRENNUNG EINES C4-SCHNITTES DURCH EXTRAKTIVDESTILLATION MIT EINEM SELEKTIVEN LÖSUNGSMITTEL**
METHOD FOR SEPARATING A C4 FRACTION BY MEANS OF EXTRACTIVE DISTILLATION USING A SELECTIVE SOLVENT
PROCEDE DE SEPARATION D'UNE FRACTION EN C4 PAR DISTILLATION EXTRACTIVE AVEC UN SOLVANT SELECTIF

(30) Priorität: 12.07.2006 EP 06117007
(43) Veröffentlichungstag der Anmeldung: 08.04.2009
(73) Patentinhaber: BASF SE, 67056 Ludwigshafen (DE)
(72) Erfinder: HEIDA, Bernd, 67158 Ellerstadt (DE); GAFFRON, Eberhardt, 68307 Mannheim (DE); SCHÄFER, Gerhard, 68229 Mannheim (DE)
(86) Internationale Anmeldenummer: PCT/EP2007/057184
(87) Internationale Veröffentlichungsnummer: WO 2008/006879

(56) Entgegenhaltungen:
- WO-A-03/093202
- WO-A-2005/075388
- GB-A- 1 564 680

## Beschreibung

Die Erfindung betrifft ein Verfahren zur Auftrennung eines C₄-Schnittes durch Extraktivdestillation mit einem selektiven Lösungsmittel.

Der beispielsweise in Crackern anfallende so genannte C₄-Schnitt umfasst ein Gemisch von Kohlenwasserstoffen, wobei die C₄-Kohlenwasserstoffe, insbesondere 1-Buten, i-Buten sowie 1,3-Butadien überwiegen. Neben geringen Menge an C3- und C₅-Kohlenwasserstoffen enthält der C₄-Schnitt in der Regel C₃- und C₄-Acetylene, z.B. 1-Butin, Butenin und Propin, insbesondere 1-Butin (Ethylacetylen) und Butenin (Vinylacetylen).

Die Gewinnung von 1,3-Butadien aus derartigen Gemischen ist wegen der geringen Unterschiede in den relativen Flüchtigkeiten ein kompliziertes destillationstechnisches Problem. Daher wird die Auftrennung durch eine so genannte Extraktivdestillation durchgeführt, das heißt eines Destillation unter Zugabe eines selektiven Lösungsmittels, das einen höheren Siedepunkt als das aufzutrennende Gemisch aufweist und das die Unterschiede in den relativen Flüchtigkeiten der aufzutrennenden Komponenten erhöht.

Durch Auswahl geeigneter selektiver Lösungsmittel ist es möglich, den C₄-Schnitt per Extraktivdestillation in einen Kopfstrom aufzutrennen, enthaltend die weniger als 1,3-Butadien löslichen Kohlenwasserstoffe, insbesondere Butane und Butene, sogenanntes Raffinat I, und ein mit den übrigen Kohlenwasserstoffen aus dem C₄-Schnitt, insbesondere 1,3-Butadien, den Butinen sowie gegebenenfalls 1,2-Butadien beladenes Lösungsmittel, woraus die genannten Kohlenwasserstoffe anschließend unter Rückgewinnung des Lösungsmittels ausgegast werden.

Die Kapazität bestehender Anlagen zur Extraktivdestillation ist insbesondere durch die folgenden Parameter festgelegt:
- die Temperatur des selektiven Lösungsmittels bei der Zuführung in die Extraktivdestillationskolonne,
- die Temperatur am Kopf der Kolonne aus der Raffinat I abgezogen wird,
- die Anzahl der theoretischen Trennstufen in der Extraktivdestillationskolonne,
- die Dampf- und/oder Flüssigkeitsbelastung der Extraktivdestillationskolonne,
- der Druck in der Extraktivdestillationskolonne sowie
- die Lösungsmittel-Umlaufmenge.

Diese Parameter können nur begrenzt im Sinne einer Steigerung der Kapazität der Anlage verändert werden, weil technische oder wirtschaftliche Grenzen erreicht werden.

So ist die Temperatur, auf die das selektive Lösungsmittel vor der Zuführung desselben abgekühlt werden kann und die Kondensationstemperatur des Kopfstromes für Raffinat I durch das verfügbare Kühlmittel, in der Regel Flusswasser, begrenzt. Die Anzahl der theoretischen Trennstufen für eine gegebene Extraktivdestillationskolonne ist bei bestehenden Anlagen vorgegeben und bei Neuanlagen aus insbesondere wirtschaftlichen Gründen begrenzt. Schließlich kann die Dampf- und/oder Flüssigkeitsbelastung einer Kolonne nicht über einen bestimmten Bereich erhöht werden, ohne den ordnungsgemäßen Betrieb derselben nachteilig zu beeinflussen.

Dokument WO-A 2005/075388, beschreibt ein Verfahren zur Gewinnung von Roh-1,3-Butadien durch Extraktivdestillation mit einem selektiven Lösungsmittel aus einem C₄-Schnitt in einer Trennwandkolonne in kompressorloser Fahrweise, das insbesondere weniger Fouling in den Kolonnen, eine erhöhte Betriebssicherheit und Wirtschaftlichkeit gewährleistet, indem man aus der Trennwandkolonne einen Sumpfstron abzieht, enthaltend mit den C₄-Acetylen beladenes Lösungsmittel, dessen Anteil an 1,3-Butadien so begrenzt ist, dass der Verlust an 1,3-Butadien wirtschaftlich akzeptabel ist und indem man den Sumpfstrom einem Acetylene-Ausgaser zuführt und im Acetylene-Ausgaser die C₄-Acetylene über Kopf ausstrippt und gereinigtes Lösungsmittel als Sumpfstrom gewinnt.

Es war daher Aufgabe der Erfindung, ein Verfahren zur Verfügung zu stellen, wonach die Kapazität bestehender Anlagen zur Auftrennung von C₄-Schnitten durch Extraktivdestillation mit einem selektiven Lösungsmittel ohne weitere Veränderung der oben dargelegten bekannten Einflussparameter gesteigert werden kann. Insbesondere sollte der aufzutrennende Feedstrom erhöht und/oder der spezifische- Energieverbrauch der Anlage ohne weitere Veränderung der Betriebsbedingungen erniedrigt werden.

Die Lösung besteht in einem Verfahren zur Auftrennung eines C₄-Schnittes durch Extraktivdestillation mit einem selektiven Lösungsmittel durch Gegenstromführung des C₄-Schnittes und des selektiven Lösungsmittels in flüssiger Phase in einer Destillationseinheit, unter Abtrennung eines Kopfstromes, enthaltend die Butane und die Butene aus dem C₄-Schnitt sowie eines Sumpfstromes, enthaltend das selektive Lösungsmittel und die übrigen Komponenten des C₄-Schnittes, außer den Butanen und den Butenen, aus dem in weiteren Verfahrensschritten die übrigen Komponenten des C₄-Schnittes, außer den Butanen und den Butenen ausgegast werden, das dadurch gekennzeichnet ist, dass zwischen der Abzugsstelle für den Kopfstrom und der Abzugsstelle für den Sumpfstrom Energie aus der Destillationseinheit abgezogen wird.

Es wurde gefunden, dass es möglich ist, die Kapazität bestehender Anlagen zur Extraktivdestillation von C₄-Schnitten in einfacher Weise, mit geringem apparativen und energetischen Aufwand durch Beeinflussung des Temperaturprofils zu verbessern, indem Energie aus dem Verfahren abgezogen wird, ohne dass hierbei die Endwerte des Temperaturverlaufs verändert werden.

Beilspielsweise kann durch Abziehen einer entsprechenden Menge an thermischer Energie eine Temperaturabsenkung von bis zu 10°C erreicht werden.

Die Erfindung ist nicht eingeschränkt bezüglich der konkreten Durchführung des Verfahrens zur Auftrennung eines C₄-Schnitts durch Extraktivdestillation mit einem selektiven Lösungsmittel. Insbesondere kann es sich auch um ein Verfahren handeln, bei dem zusätzlich zur Extraktivdestillation eine Selektivhydrierung acetylenischer Verunreinigungen durchgeführt wird.

Das erfindungsgemäße Verfahren ist beispielsweise anwendbar auf eine Verfahrensvariante, bei der die Destillationseinheit, in der die Extraktivdestillation durchgeführt wird, aus zwei Kolonnen gebildet ist, die untereinander mit einer Flüssigkeitsleitung und einer Dampfleitung verbunden sind. In dieser Verfahrensvariante wird vorteilhaft Energie aus der Flüssigkeits- und/oder der Dampfleitung abgezogen. In der Verfahrensvariante mit zwei Kolonnen wird der ersten Kolonne bevorzugt dem unteren Bereich derselben, der C₄-Schnitt, bevorzugt dampfförmig zugeführt und im Gegenstrom hierzu, im oberen Bereich der ersten Kolonne, das selektive Lösungsmittel. Am Kopf der ersten Kolonne wird ein Strom, enthaltend die leichter als 1,3-Butadien siedenden Kohlenwasserstoffe, insbesondere Butane und Butene, so genanntes Raffinat I, abgezogen.

Die erste Kolonne ist über eine Flüssigkeits- und eine Dampfleitung mit einer zweiten Kolonne verbunden, in der die Abtrennung eines mit den übrigen Komponenten aus dem C₄-Schnitt gegenüber den Butanen und Butenen beladenen selektiven Lösungsmittels über Sumpf erfolgt. Hieraus werden in einem oder mehreren weiteren Apparaten die weiteren Komponenten des C₄-Schnitts aus dem selektiven Lösungsmittel abgetrennt, wobei insbesondere ein Roh-1,3-Butadienstrom erhalten wird. Hierbei wird als Roh-1,3-Butadien in bekannter Weise ein Kohlenwasserstoffgemisch verstanden, das das Wertprodukt 1,3-Butadien in einem Anteil von mindestens 80 Gew.%, bevorzugt mindestens 90 Gew.%, oder auch mindestens 95 Gew.%, Rest Verunreinigungen, enthält.

Zusätzlich oder alternativ ist es möglich, an einer oder mehreren weiteren Stellen der Destillationseinheit Flüssigkeit und/oder Gas abzuziehen, über einen außen liegenden Wärmetauscher zu führen und darin abzukühlen und erneut in die Destillationseinheit an derselben Stelle zurückzuführen, an der der Abzug der Flüssigkeit oder des Gases erfolgt war, oder an einer hiervon verschiedenen Stelle.

Zusätzlich oder alternativ ist es möglich, Flüssigkeit und/oder Gas über innenliegende Kühleinrichtungen in der Destillationseinheit zu kühlen.
Darüber hinaus ist es zusätzlich oder alternativ möglich, den der Destillationseinheit zugeführten C₄-Schnitt teilweise oder vollständig in flüssiger Form zuzuführen.

Der vorliegend als Ausgangsgemisch einzusetzende sogenannte C₄-Schnitt ist ein Gemisch von Kohlenwasserstoffen mit überwiegend vier Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, Butene, 1,3-Butadien, daneben geringe Mengen an C₃- und C₅-Kohlenwasserstoffen, sowie Butine, insbesondere 1-Butin (Ethylacetylen) und Butenin (Vinylacetylen). Dabei beträgt der 1,3-Butadiengehalt im Allgemeinen 10 bis 80 Gew.-%, vorzugsweise 20 bis 70 Gew.-%, insbesondere 30 bis 60 Gew.-%, während der Gehalt an Vinylacetylen und Ethylacetylen im Allgemeinen 5 Gew.-% nicht übersteigt.

Geeignete selektive Lösungsmittel für das erfindungsgemäße Verfahren sind zum Beispiel Butyrolacton, Nitrile wie Acetonitril (ACN), Propionitril, Methoxypropionitril, Ketone wie Aceton, Furfural, N-alkylsubstituierte niedere aliphatische Säureamide, wie Dimethylformamid (DMF), Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im Allgemeinen werden N-alkylsubstituierte niedere aliphatische Säureamide oder N-alkylsubstituierte cyclische Säureamide verwendet. Besonders vorteilhaft sind Dimethylformamid und insbesondere N-Methylpyrrolidon.

Es können jedoch auch Mischungen dieser Lösungsmittel untereinander, zum Beispiel von N-Methylpyrrolidon mit Acetonitril, sowie Mischungen dieser Lösungsmittel mit Colösungsmitteln, wie Wasser, Ethern, zum Beispiel Methyl-tert.-butylether, Ethyl-tert.-butylether, Propyl-tert.-butylether, n- oder iso-Butyl-tert.-butylether und/oder Alkoholen, zum Beispiel Isopropanol, eingesetzt werden.

Besonders geeignet ist N-Methylpyrrolidon, bevorzugt in wässriger Lösung, insbesondere mit 8 bis 10 Gew.-% Wasser, besonders bevorzugt mit 8,3 Gew.-% Wasser.

Besonders geeignet sind auch DMF, ACN, sowie Mischungen von ACN mit Wasser und/oder Alkohol.

Die Erfindung wird im Folgenden anhand einer Zeichnung und eines Ausführungsbeispiels näher erläutert.

Es zeigen im Einzelnen:
- Figur 1: die schematische Darstellung einer Destillationseinheit mit zwei Kolonnen KI und KII, an die sich eine Reinigungs- und Entgasungseinheit E anschließt.
- Figur 2: die schematische Darstellung des Temperaturverlaufs in der in Figur 1 dargestellten Anlage, ohne Abzug von Energie (Kurve I, zum Vergleich) und mit Abzug von Energie zwischen der Abzugsstelle für den Kopfstrom und der Abzugsstelle für den Sumpfstrom (Kurve II, erfindungsgemäß).

Einer Destillationseinheit, umfassend zwei Destillationskolonnen KI und KII wird ein selektives Lösungsmittel, Strom 1, bei einer Temperatur T1 im oberen Bereich der Kolonne KI zugeführt, und ein aufzutrennender C₄-Schnitt, Strom 2, im unteren Bereich der Kolonne KI. Aus der Kolonne KI wird ein Kopfstrom 3 abgezogen, in einem außen liegenden Kondensator auf eine Temperatur T2 abgekühlt, teilweise aus dem Verfahren abgezogen und im übrigen als Rücklauf auf der Kolonne KI aufgegeben.

Die Kolonne KI ist über eine Flüssigkeitsleitung 4 und eine Dampfleitung 5 mit der Kolonne KII verbunden. Aus der Kolonne KII wird ein Sumpfstrom 6 abgezogen, enthaltend selektives Lösungsmittel, das mit den restlichen Komponenten des C₄-Schnitts außer den Butanen und Butenen beladen ist. Dieser Strom wird einer Reinigungs- und Entgasungseinheit E zugeführt und hieraus ein Roh-1,3-butadien enthaltender Strom 9 sowie ein C₄-Acetylene sowie Schwersieder, das heißt Verbindungen mit einem Siedepunkt höher als dem Siedepunkt von 1,3-Butadien, enthaltender Strom 10, abgezogen, unter Verbleib von gereinigtem selektivem Lösungsmittel, das als Sumpfstrom 8 abgezogen wird.

Die Reinigungs- und Entgasungseinheit E ist über eine Dampfleitung 7 mit der Kolonne KII verbunden.

Erfindungsgemäß wird aus den Verbindungsleitungen 4 und/oder 5 zwischen den Kolonnen KI und KII, von einer oder mehreren geeigneten Stellen der Kolonnen KI und/oder KII und/oder aus dem Feedstrom enthaltend den C₄-Schnitt, Strom 2, Energie abgezogen.

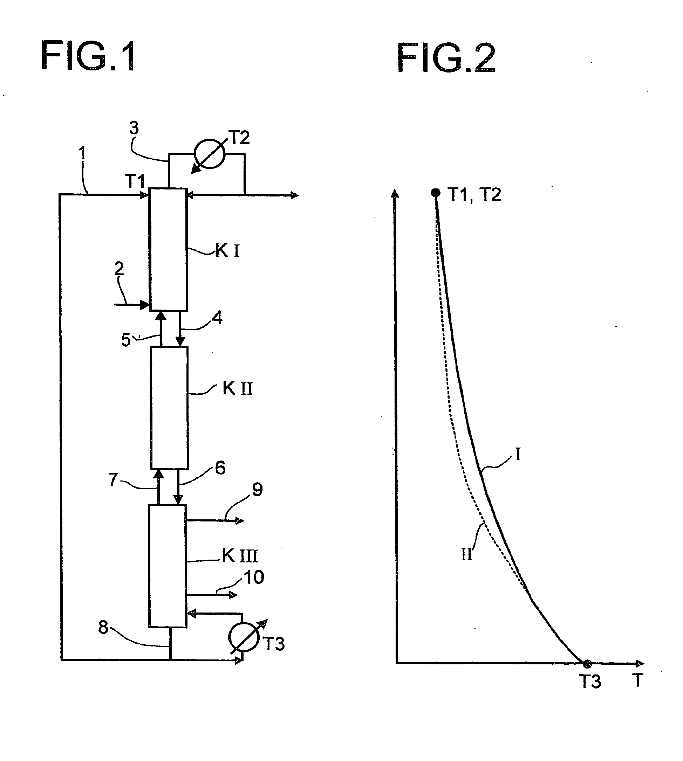

Figur 2 zeigt die schematische Darstellung des Temperaturverlaufs in einem Verfahren nach dem Stand der Technik (Kurve I) ohne Abzug von Energie, und in einem Verfahren nach der Erfindung (Kurve II) mit Abzug von Energie zwischen den Abzugsstellen für den Kopfstrom 3 und den Sumpfstrom 6.

### Ausführungsbeispiel

In einem Verfahren zur Auftrennung eines C4-Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wurde der Flüssigkeitsstrom 4 aus der Kolonne KI abgezogen, um 10°C abgekühlt und in das Verfahren an der Abzugsstelle desselben recycliert. Dadurch konnte die Menge des Feedstromes, enthaltend den C₄-Schnitt Strom 2, um 8 % gegenüber dem Verfahren zum Vergleich, ohne Abkühlung des Stromes 4, um 8 % erhöht werden, bei ansonsten unveränderten Betriebsbedingungen, und die Kapazität der Anlage entsprechend erhöht werden.

## Patentansprüche

1. Verfahren zur Auftrennung eines C₄-Schnittes (2) durch Extraktivdestillation mit einem selektiven Lösungsmittel (1) durch Gegenstromführung des C4-Schnittes und des selektiven Lösungsmittels (1) in flüssiger Phase in einer Destillationseinheit (KI, KII) unter Abtrennung eines Kopfstromes (3), enthaltend die Butane und die Butene aus dem C₄-Schnitt sowie eines Sumpfstromes (6), enthaltend das selektive Lösungsmittel und die übrigen Komponenten des C₄-Schnittes, außer den Butanen und den Butenen, aus dem in weiteren Verfahrensschritten die übrigen Komponenten des C4-Schnittes, außer den Butanen und den Butenen ausgegast werden, **dadurch gekennzeichnet, dass**
zwischen der Abzugsstelle für den Kopfstrom (3) und der Abzugsstelle für den Sumpfstrom (6) Energie aus der Destillationseinheit (KI, KII) abgezogen wird.

2. Verfahren nach Anspruch 1, **dadurch gekennzeichnet, dass** eine Energiemenge abgezogen wird, die einer nach Temperaturabsenkung von bis zu 10°C entspricht.

3. Verfahren nach Anspruch 1 oder 2, **dadurch gekennzeichnet, dass** die Destillationseinheit zwei Kolonnen (KI, KII) umfasst, die untereinander mit einer Flüssigkeitsleitung (4) und einer Dampfleitung (5) verbunden sind und dass Energie aus der Flüssigkeitsleitung (4) und/oder der Dampfleitung (5) abgezogen wird.

4. Verfahren nach einem der Ansprüche 1 bis 3, **dadurch gekennzeichnet, dass** an einer oder mehreren Stellen aus der Destillationseinheit (KI, KII) Flüssigkeit und/oder Gas abgezogen, in einem Außen liegenden Kühler abgekühlt und in die Destillationseinheit (KI, KII) an derselben Stelle oder einer von der Stelle des Abzugs der Flüssigkeit und/oder des Gases verschiedenen Stelle zurückgeführt wird.

5. Verfahren nach einem der Ansprüche 1 bis 4, **dadurch gekennzeichnet, dass** Flüssigkeit und/oder Dampf über einen innen liegenden Kühler in der Destillationseinheit (KI, KII), der zwischen der Abzugsstelle für den Kopfstrom (3) und der Abzugsstelle für den Sumpfstrom (6) angeordnet ist, geleitet wird.

6. Verfahren nach einem der Ansprüche 1 bis 5, **dadurch gekennzeichnet, dass** der C₄-Schnitt der Destillationseinheit (KI, KII) ganz oder teilweise flüssig zugeführt wird.

7. Verfahren nach einem der Ansprüche 1 bis 6, **dadurch gekennzeichnet, dass** als selektives Lösungsmittel N-Methylpyrrolidon, bevorzugt in wässriger Lösung, insbesondere mit 8 bis 10 Gew.% Wasser, besonders bevorzugt mit 8,3 Gew.% Wasser, eingesetzt wird.

8. Verfahren nach einem der Ansprüche 1 bis 6, **dadurch gekennzeichnet, dass** als selektives Lösungsmittel Dimethylformamid eingesetzt wird.

9. Verfahren nach einem der Ansprüche 1 bis 6, **dadurch gekennzeichnet, dass** als selektives Lösungsmittel Acetonitril oder Mischung hiervon mit Wasser und/oder einem oder mehreren Alkoholen, eingesetzt wird.

## Claims

1. A process for separating a C₄ cut (2) by extractive distillation with a selective solvent (1) by countercurrent flow of the C4 cut and of the selective solvent (1) in the liquid phase in a distillation unit (KI, KII) with removal of a top stream (3) comprising the butanes and the butenes from the C₄ cut, and a bottom stream (6) comprising the selective solvent and the remaining components of the C₄ cut apart from the butanes and the butenes, from which, in further process steps, the remaining components of the C4 cut apart from the butanes and the butenes are outgassed, wherein energy is drawn off from the distillation unit (KI, KII) between the draw point for the top stream (3) and the draw point for the bottom stream (6).

2. The process according to claim 1, wherein an amount of energy which corresponds to a temperature decrease of up to 10°C is drawn off.

3. The process according to claim 1 or 2, wherein the distillation unit comprises two columns (KI, KII) which are connected to one another with a liquid line (4) and a vapor line (5), and energy is drawn off from the liquid line (4) and/or the vapor line (5).

4. The process according to any of claims 1 to 3, wherein liquid and/or gas is drawn off at one or more points from the distillation unit (KI, KII), cooled in an external cooler and recycled into the distillation unit (KI, KII) at the same point or at a different point from the point at which the liquid and/or the gas is drawn off.

5. The process according to any of claims 1 to 4, wherein liquid and/or vapor is passed through an internal cooler in the distillation unit (KI, KII), which is arranged between the draw point for the top stream (3) and the draw point for the bottom stream (6).

6. The process according to any of claims 1 to 5, wherein the C₄ cut of the distillation unit (KI, KII) is fed completely or partly in liquid form.

7. The process according to any of claims 1 to 6, wherein the selective solvent used is N-methylpyrrolidone, preferably in aqueous solution, in particular with from 8 to 10% by weight of water, more preferably with 8.3% by weight of water.

8. The process according to any of claims 1 to 6, wherein the selective solvent used is dimethylformamide.

9. The process according to any of claims 1 to 6, wherein the selective solvent used is acetonitrile or a mixture thereof with water and/or one or more alcohols.

## Revendications

1. Procédé pour la séparation d'une fraction en C₄ (2) par distillation extractive avec un solvant sélectif (1) par conduite à contre-courant de la fraction en C₄ et du solvant sélectif (1) en phase liquide dans une unité de distillation (KI, KII) avec séparation d'un courant de tête (3), contenant les butanes et les butènes provenant de la fraction en C₄, ainsi que d'un courant de pied (6), contenant le solvant sélectif et les composants restants de la fraction en C₄, hormis les butanes et les butènes, à partir duquel dans d'autres étapes du procédé les autres composants de la fraction en C₄, hormis les butanes et les butènes, sont évacués sous forme de gaz, **caractérisé**
**en ce qu'**entre le point d'évacuation du courant de tête (3) et le point d'évacuation du courant de pied (6) de l'énergie est évacuée de l'unité de distillation (KI, KII).

2. Procédé selon la revendication 1, **caractérisé en ce qu'**est évacuée une quantité d'énergie qui correspond à une quantité après abaissement de la température de jusqu'à 10 °C.

3. Procédé selon la revendication 1 ou 2, **caractérisé en ce que** l'unité de distillation comprend deux colonnes (KI, KII) qui sont reliées l'une à l'autre par un conduit pour liquides (4) et un conduit pour vapeurs (5) et **en ce que** de l'énergie est évacuée du conduit pour liquides (4) et/ou du conduit pour vapeurs (5).

4. Procédé selon l'une quelconque des revendications 1 à 3, **caractérisé en ce qu'**un liquide et/ou un gaz est/sont évacué (s) en un ou plusieurs points de l'unité de distillation (KI, KII), refroidi(s) dans un refroidisseur externe et renvoyé(s) dans l'unité de distillation (KI, KII) en le même point ou en un point différent du point de l'évacuation du liquide et/ou du gaz.

5. Procédé selon l'une quelconque des revendications 1 à 4, **caractérisé en ce qu'**on fait passer le liquide et/ou la vapeur sur un refroidisseur interne se trouvant dans l'unité de distillation (KI, KII), qui est disposé entre le point d'évacuation pour le courant de tête (3) et le point d'évacuation pour le courant de pied (6).

6. Procédé selon l'une quelconque des revendications 1 à 5, **caractérisé en ce que** la fraction en C₄ de l'unité de distillation (KI, KII) est introduite en partie ou en totalité à l'état liquide.

7. Procédé selon l'une quelconque des revendications 1 à 6, **caractérisé en ce qu'**on utilise comme solvant sélectif la N-méthylpyrrolidone, de préférence en solution aqueuse, en particulier avec 8 à 10 % en poids d'eau, de façon particulièrement préférée avec 8,3 % en poids d'eau.

8. Procédé selon l'une quelconque des revendications 1 à 6, **caractérisé en ce qu'**on utilise comme solvant sélectif le diméthylformamide.

9. Procédé selon l'une quelconque des revendications 1 à 6, **caractérisé en ce qu'**on utilise comme solvant sélectif l'acétonitrile ou un mélange de celui-ci avec l'eau et/ou un ou plusieurs alcools.
